# EUROPEAN PATENT APPLICATION

(11) **EP 2 133 052 A1**
(43) Date of publication of application: **16.12.2009**
(21) Application number: 08738718.9
(22) Date of filing: 21.03.2008
(51) Int. Cl.: A61F 13/15, A61F 13/534

(54) **ABSORPTIVE ARTICLE**

(30) Priority: 23.03.2007 JP 2007076164
(71) Applicant: Unicharm Corporation, Ehime 799-0111 (JP)
(72) Inventor: NODA, Yuki, Kanonji-shi Kagawa 769-1602 (JP); KURODA, Kenichiro, Kanonji-shi Kagawa 769-1602 (JP); NISHIKAWA, Kumiko, Kanonji-shi Kagawa 769-1602 (JP)
(74) Representative: Sperling, Rüdiger
(86) International application number: PCT/JP2008/055325
(87) International publication number: WO 2008/117763

(57) **Abstract**

An absorptive article has an absorption body provided with a bending section formed at its predetermined position, and this allows the absorptive article to deform along the shape of an object to which the article is attached. A sanitary napkin (1), as the absorptive article has a liquid permeable front side sheet (2), a liquid impermeable rear side sheet (3), and the absorption body (4) placed between the front side sheet (2) and the rear side sheet (3) and having the bending section formed at the predetermined position. When applied to the human body, the sanitary napkin (1) deforms along its shape.

## Description

### TECHNICAL FIELD

The present invention relates to an absorbent article.

### BACKGROUND ART

Conventionally, various improvements have been made to absorbent articles such as sanitary napkins, to allow bending along the shape of a wearer's body to prevent excretory substances such as menstrual blood from leaking out.

For example, an absorbent article with a three-dimensional groove formed on the skin-contact face of the absorbent article, the groove disposed from a central portion in the longitudinal direction of the absorbent article to a rear portion placed on the rear side of the wearer when the article is worn. The three dimensional groove comes closer to a side area of the rear portion as approaching the bottom edge. The three dimensional groove is disposed to come between the rear end edge and the side edge, and includes a convex portion projecting to the front side in the central portion of the longitudinal direction (for example, refer to Patent Document 1).
Patent Document 1: Japanese Patent No. 3566012

### DISCLOSURE OF THE INVENTION

### Problems to be Solved by the Invention

The absorbent article disclosed by Patent Document 1 includes a three-dimensional groove in the central portion forming the convex portion projecting to the front side. Further, an absorbent article with a three-dimensional groove is disclosed that forms the three-dimensional groove by embossing, causing the density to be higher and the thickness thinner than the surrounding area, so that the absorbent article easily deforms at the three-dimensional groove as a bending axis. However, since the three-dimensional groove formed by embossing has a much higher stiffness as a tradeoff of a higher density, a greater load is required to bend the absorbent article. Therefore, for example, when everyday panties which have a weak tightning force are worn, the absorbent article does not deform, so that the absorbent article does not fit the wearer's body, thereby causing a feeling of a foreign body, or leakage of menstrual blood due to a gap between the absorbent article and the wearer's body.

The object of the present invention is to provide an absorbent article which can be deformed along with a shape of an intended wearer by comprising an absorbent article formed with bending portions at predetermined positions.

### Means for Solving the Problems

In a first aspect of the present invention, an absorbent article having a width direction and a longitudinal direction orthogonal to the width direction, and a front edge portion and a rear edge portion in the longitudinal direction, including: a surface sheet such that at least a portion is liquid permeable; a back sheet which is liquid impermeable; and an elongated shaped absorbent body disposed between the surface sheet and the back sheet along the longitudinal direction, in which the absorbent body has a bending portion with a bending stiffness lower than an average bending stiffness of the absorbent body and lower than a bending stiffness of neighboring regions thereof, and in which the bending portion includes: a front bending portion disposed at a front portion in the longitudinal direction, formed to extend from a central side in the width direction to an external side in the width direction as the front bending portion extends from the front edge portion side to a central side in the longitudinal direction; a rear bending portion disposed at a rear portion in the longitudinal direction, formed to extend from the central side in the width direction to the external side in the width direction as the rear bending portion extends from the rear edge portion side to the central side in the longitudinal direction; and a first bending portion formed between the rear bending portion and the rear edge portion, formed to extend from the external side in the width direction to the central side in the width direction, as the first bending portion extends from the rear edge portion side to the central side in the longitudinal direction.

In a second aspect of the absorbent article as described in the first aspect of the present invention, in which the absorbent body further includes: a first region which can be raised toward the surface sheet side while the absorbent body is in the worn state, formed between the rear bending portion and the first bending portion; and a second bending portion formed in the first region.

In a third aspect of the absorbent article as described in the first and the second aspect of the present invention, in which the bending portion further includes a third bending portion formed at both sides of a first center line dividing equally the absorbent body in the width direction, extending in the longitudinal direction between the first bending portion and the rear edge portion.

In a fourth aspect of the absorbent article as described in any one of the first through the third aspect of the present invention, in which the bending portion further includes: a fourth bending portion formed to extend from the central side in the width direction to the external side in the width direction as the fourth bending portion extends from the front edge portion side to the central side in the longitudinal direction between the front bending portion and the front edge portion.

In a fifth aspect of the absorbent article according to any one of the first through the fourth aspects of the present invention, in which the bending portion is formed substantially symmetrically with respect to the first center line.

### Effects of the Invention

The present invention is to provide an absorbent article which can be deformed along with a shape of an intended wearer by comprising an absorbent article formed with bending portions at predetermined positions.

### BRIEF DESCRIPTION OF THE DRAWINGS

Fig. 1 is a top view of a sanitary napkin 1 according to a first embodiment of the present invention;
Fig. 2 is a partial cross-sectional view of a sanitary napkin 1;
Fig. 3 is a top view of an absorbent body 4 constituting the sanitary napkin 1;
Fig. 4A is a cross-sectional view in the longitudinal direction of the absorbent body 4;
Fig. 4B is a cross-sectional view in the lateral direction of the absorbent body 4;
Fig. 5 is a perspective view illustrating a deformed state of the absorbent body 4 in a state when the sanitary napkin 1 is worn;
Fig. 6 is a diagram illustrating a manufacturing device for manufacturing the absorbent body 4; and
Fig. 7 is a perspective view illustrating a structure in a meshed case 200 constituting the manufacturing device.

### PREFERRED MODE FOR CARRYING OUT THE INVENTION

Preferred embodiment of the present invention will be described hereinafter with reference to the drawings.

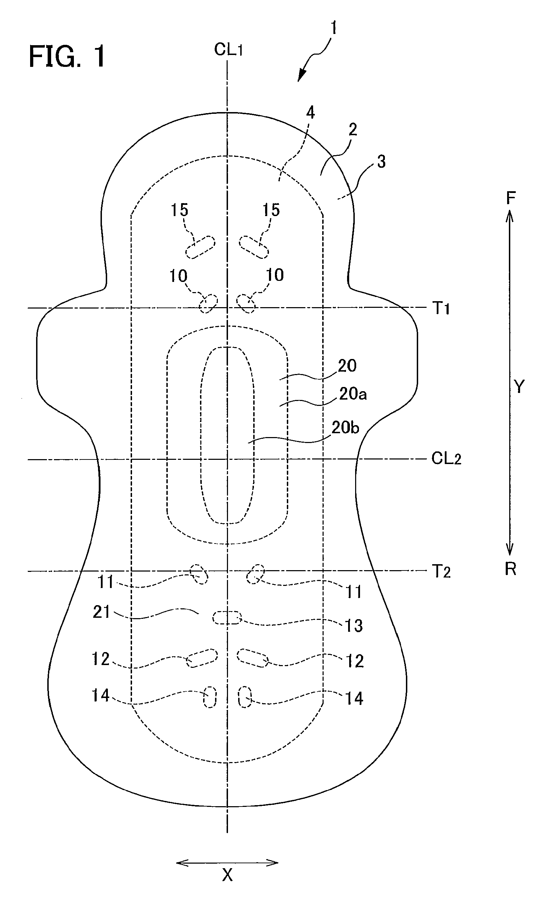

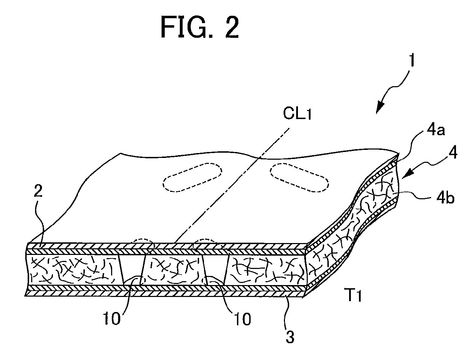

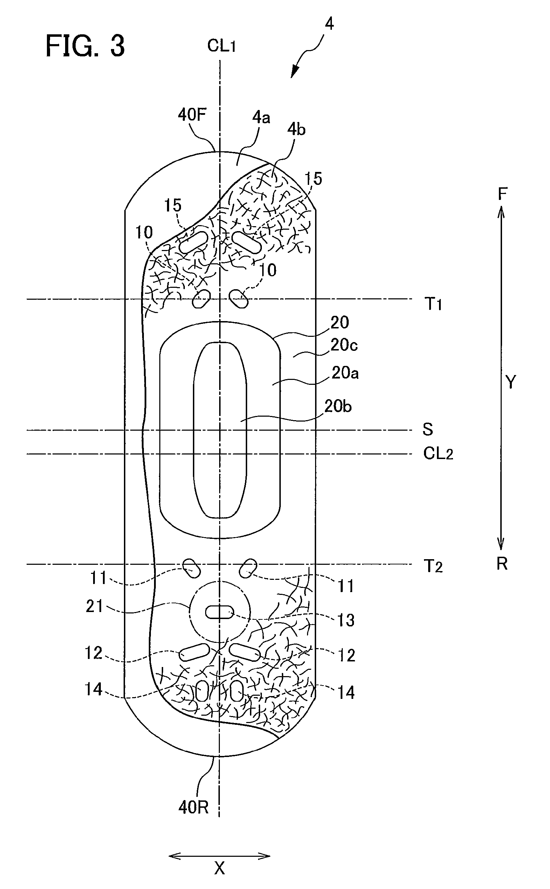

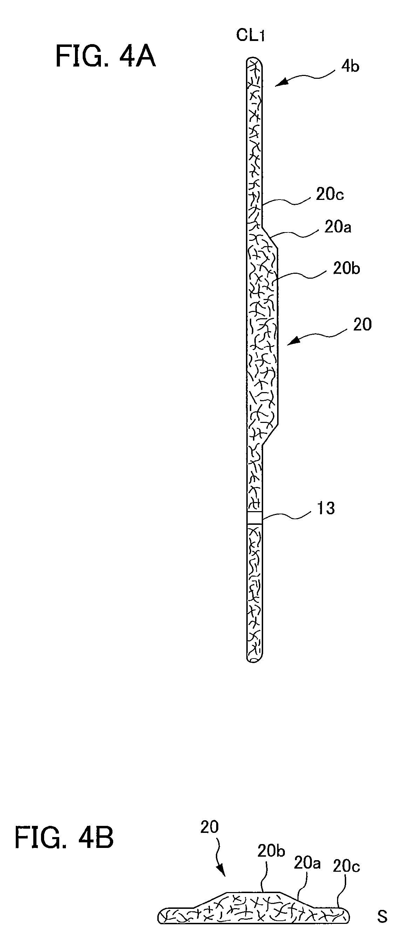

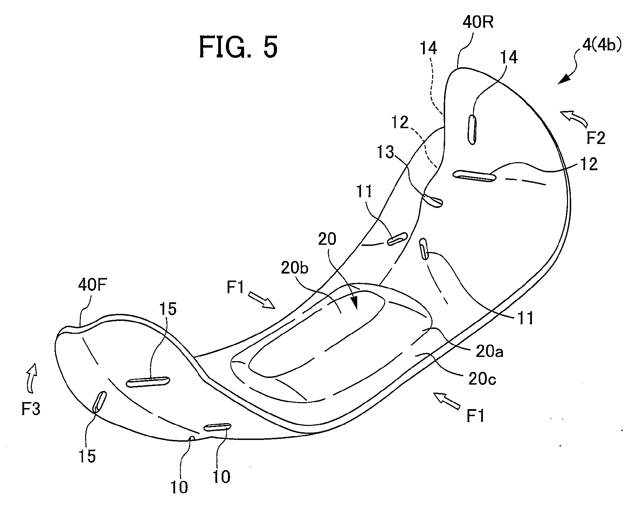

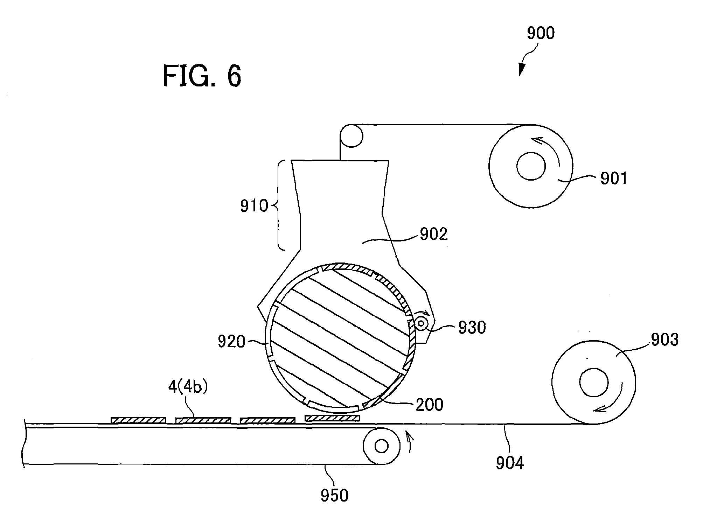

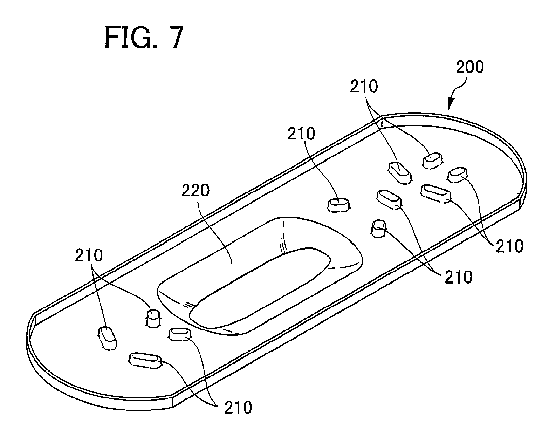

Fig. 1 is a top view of a sanitary napkin 1 according to an embodiment of the present invention. Fig. 2 is a partial cross-sectional view of a sanitary napkin 1. Fig. 3 is a top view of an absorbent body 4 constituting the sanitary napkin 1. Figs. 4A and 4B are cross-sectional views of the absorbent body 4. Fig. 5 is a perspective view of a deformed state of the absorbent body 4 when the sanitary napkin 1 is worn. Fig. 6 is a diagram illustrating a manufacturing device for manufacturing the absorbent body 4. Fig. 7 is a perspective view illustrating a structure of a meshed case 200 constituting the manufacturing device.

The sanitary napkin 1 as the absorbent article of the present invention is described with reference to Figs. 1 to 7.

### 1. Overall Configuration

As shown in Fig. 1 and Fig. 2, the sanitary napkin 1 according to the present embodiment comprises a surface sheet 2 of which at least a portion is liquid-permeable, a liquid-impermeable back sheet 3, and an absorbent body 4 of vertically long shape disposed between the surface sheet 2 and the back sheet 3 along the longitudinal direction of the sanitary napkin 1. The sanitary napkin 1 has a flat and elongated shape.

The sanitary napkin 1 viewed from one side (top face shown in Fig. 1) has basically a calabash shape, in other words a substantially oval shape with a central portion in a longitudinal direction being concave toward a central side in a width direction and a rear portion of the sanitary napkin being wider than a front portion, and has a wing on both outer edges in a width direction X, in a front side F of a longitudinal direction Y. Although the sanitary napkin 1 has the aforementioned shape in the present embodiment, it may have any shape suitable for the shape of women's body and panties; for example, a rectangle shape, an elliptical shape, and the like. It should be noted that, the front portion is a region at a side of the front edge portion, which is one of regions divided into two in the longitudinal direction, preferably in the region of the front side F of the vicinity of T1 of Fig. 1, and the rear portion is a region at a side of the rear edge portion, which is another of regions divided into two in the longitudinal direction, preferably in the region of the rear side R of the vicinity of T2 of Fig. 1.

The length of the sanitary napkin 1 in the longitudinal direction Y is from 100 to 500 mm, and preferably from 150 to 350 mm. Also, the length of the sanitary napkin 1 in the width direction (lateral direction) X is from 30 to 200 mm, and preferably from 40 to 180 mm. Here, the surface sheet 2 and the back sheet 3 will later be described in detail. In addition, the absorbent body 4 will be described in detail hereinafter.

### 2. Absorbent Body

The absorbent body 4 in the sanitary napkin 1 will be described with reference to Figs. 1 to 7.

As shown in Fig. 1 and Fig. 3, the absorbent body 4 is constituted of a core portion 4b and a covering sheet 4a which covers the core portion 4b. In addition, the absorbent body 4 has a substantially flat and a vertically long shape.

### 2.1. Bending Portions

The absorbent body 4 has bending portions. The bending portions are: a front bending portion 10, 10; a rear bending portion 11, 11; a first bending portion 12, 12; a second bending portion 13; a third bending portions 14, 14; and a fourth bending portions 15, 15. The bending portions may be, for example, one closed region like in the second bending portion 13, and may include a pair of or a plurality of the closed region like in the front and rear bending portions 10, 10; 11, 11; and the first, third, and fourth bending portions 12, 12; 14, 14; and 15, 15. Each of the front bending portions 10, 10; the rear bending portions 11, 11; the first bending portions 12, 12; the second bending portion 13; the third bending portions 14, 14; and the fourth bending portions 15, 15 are regions having a bending stiffness lower than an average bending stiffness of the absorbent body 4, and lower than a bending stiffness of neighboring regions thereof. The front bending portion 10, 10 the rear bending portion 11, 11 the first bending portion 12, 12 the second bending portion 13, 13 the third bending portion 14, 14 and the fourth bending portion 15, 15 are formed to have a weight lower than an average weight of the absorbent body 4 and than a weight of neighboring regions thereof. For example, a weight of each bending portion is preferably not higher than 80% of a weight of neighboring regions, more preferably in the range of 0 to 80% thereof, even more preferably in the range of 5 to 30% thereof. Specifically, as shown in Fig. 2, the bending portions are regions in which an absorbent material constituting the core portion 4b is not substantially disposed.

In addition, each of the front bending portions 10, 10; the rear bending portions 11, 11; the first bending portions 12, 12; the second bending portion 13; the third bending portions 14, 14; and the fourth bending portions 15, 15 constituting these bending portions are formed to be symmetrical with respect to the first center line CL1 equally dividing the absorbent body 4 in the width direction X.

Here, a length (longitudinal dimension) in each of the bending portions is preferably in the range of 5 to 50 mm and more preferably in the range of 10 to 30 mm; and a width (lateral dimension) thereof is preferably in the range of 1 to 10 mm and more preferably in the range of 2 to 5 mm. Also, regarding a planar shape of each of the bending portions, a cross section in a planar direction has a rectangular shape and a cross section in a direction vertical to the planar direction has a tapered trapezoidal shape. Here, in the present embodiment, the bending portions have the aforementioned shape, but need not be limited to this; for example, a cross section thereof in a planar direction may have, for example, a circular shape, an elliptical shape, a square shape, and a triangle shape.

Additionally, a surface area of the bending portions (a surface area of a side of the surface sheet 2) is at least 5 mm², more preferably in the range of 5 to 500 mm², and even more preferably in the range of 50 to 200 mm². A surface area ratio of the bending portions is at least 5% to a total surface area of the absorbent body 4, preferably in the range of 5 to 50%, and even more preferably in the range of 10 to 40%. By setting each of the bending portions to the aforementioned dimension and the like, the absorbent body can be deformed along with a shape of an intended wearer, while restraining deformation of a shape which does not accommodate the intended wearer.

Each of the front bending portions 10, 10 is formed on both sides in the width direction X of the first center line CL1, being symmetrical with respect to the first center line CL1. Each of the front bending portions 10, 10 is formed to extend from a central side in the width direction X to an external side in the width direction X as the front bending portions 10, 10 extend from a front edge portion 40F side of the absorbent body 4 to a central side in the longitudinal direction Y.

The rear bending portions 11, 11 are formed nearer to a rear edge portion 40R of the absorbent body 4 than the front bending portion 10, 10. Additionally, each of the rear bending portions 11, 11 is formed on both sides in the width direction X of the first center line CL1, being symmetrical with respect to the first center line CL1. Also, the rear bending portions 11, 11 are formed to extend from the central side in the width direction X to the external side in the width direction X as the rear bending portions 11, 11 extend from the rear edge portion 40R side to the central side in the longitudinal direction Y.

The first bending portions 12, 12 are formed on both sides in the width direction X of the first center line CL1, being symmetrical with respect to the first center line CL1. The first bending portions 12, 12 are formed between the rear bending portions 11, 11 extending from the external side in the width direction X to the central side in the width direction X as the first bending portions 12, 12 extend from the rear edge portion 40R side to the central side in the longitudinal direction Y.

The second bending portion 13 is formed between the front bending portions 10, 10 and the first bending portions 12, 12, and formed to cross substantially orthogonal to the first center line CL1. In other words, the second bending portion 13 is formed in a first region 21 (described later) in a substantially center portion of the absorbent body 4 in the width direction X, extending toward both outer edges in the width direction X.

Each of third bending portions 14, 14 are formed between the first bending portions 12, 12, and the rear edge portion 40R. Additionally, the third bending portions 14, 14 are formed on both sides of the first center line CL1, extending in the longitudinal direction Y.

### 2.2. First Region and Second Region

The absorbent body 4 has a first region 21 which is formed at a side of the rear edge portion 40R in the longitudinal direction Y, and a second region 20 which is formed in the central side in the longitudinal direction Y.

The first region 21 is formed between the rear bending portions 11, 11 and the first bending portions 12, 12 in the longitudinal direction Y. Additionally, as stated above, the second bending portion 13, is formed to cross the first center line CL1 and extend in the width direction X.

The first region 21 is formed so as to be substantially planar when the sanitary napkin 1 is not worn (in a flattened state). Furthermore, the first region 21 is deformed so as to be raised toward the surface sheet 2 (a top face side shown in Fig. 3) when the sanitary napkin 1 is worn (in a deformed state).

The second region 20 is formed in the central portion in the longitudinal direction Y of the absorbent body 4. Specifically, the second region 20 is formed in the absorbent body 4, between the front bending portions 10, 10 and the rear bending portions 11, 11.

As shown in Figs. 3, 4A and 4B, the second region 20 has a slope 20a and a projecting portion 20b.

A slope 20a is a portion raised at a predetermined angle from a planar portion 20c toward a surface sheet 2 side. Specifically, the slope 20a slants upward toward the surface sheet 2 side, from the external side to the central side in the width direction X, and from an outer side to a central side in the longitudinal direction Y.

Furthermore, the projecting portion 20b is formed in a position surrounded by the slope 20a. The projecting portion 20b is a portion projecting more toward the surface sheet 2 side than does the planar portion 20c, and the top face thereof is formed to be substantially planar.

Here, as shown in Figs. 4A and 4B, the second region 20 is raised (projected) toward the surface sheet 2 side (the top face shown in Fig. 3) when the sanitary napkin 1 is not worn (in a flattened state). In addition, the second region 20 is further raised toward the surface sheet 2 side when the sanitary napkin 1 is worn (in a deformed state) than when the sanitary napkin 1 is not worn (see Fig. 5).

### 2.3. Folding Portion

The sanitary napkin 1 is constructed to be foldable with the surface sheet 2 inside. Specifically, the absorbent body 4 is deformed to be folded at a folding portion T1 and a folding portion T2, with the surface sheet 2 inside. The front bending portions 10, 10 are formed to cross the folding portion T1.
The rear bending portions 11, 11 are formed to cross the folding portion T2.

In this way, by forming the front bending portions 10, 10 to cross the folding portion T1 and to constitute a portion of the folding portion T1, the sanitary napkin 1 is easily foldable. Moreover, in the case that the folded sanitary napkin 1 is opened to flattened state, since a bending retention due to a cellulosic fiber and the like (described later) contained in the absorbent body 4 can be thus decreased, the folded sanitary napkin 1 can be easily opened out flat.

Similarly, by forming the rear bending portions 11, 11 to cross the folding portion T2 and to constitute a portion of the folding portion T2, the sanitary napkin 1 is easily foldable. Moreover, in the case that the folded sanitary napkin is opened to flattened state, since a bending retention due to a cellulosic fiber and the like (described later) contained in the absorbent body 4 can be thus decreased, the folded sanitary napkin 1 can be easily opened out flat.

### 2.4. Core Portion and Cover Sheet

The absorbent body 4 is formed by covering a core portion 4b with a cover sheet 4a. The core portion 4b includes 80 to 100% by mass of pulp and 0 to 20% by mass of particulate polymer. This core 4b is covered with a tissue, which is the covering sheet 4a, and, by embossing finish, a weight thereof is adjusted to be in the range of 100 to 2000 g/m² and a bulkiness thereof is adjusted to be in the range of 1 to 50 mm. Here, the embossing finish is applied to the absorbent body 4 to prevent the absorbent body 4 from losing its shape. The ratio of the embossing area to the total surface area of the absorbent body 4 is preferably in the range of 10% to 100%, and more preferably 30% to 80%.

The absorbent material constituting the core 4b may be, for example, a hydrophilic fiber including the aforementioned pulp. The hydrophilic fiber may be, for example, celluloses such as pulp and cotton, recycled celluloses such as rayon and fibril rayon, semi-synthetic celluloses such as acetate and triacetate, polymer absorbers such as particulate polymer and fibrous polymer, thermoplastic hydrophobic chemical fiber, and hydrophilized thermoplastic hydrophobic chemical fiber that can be used singly or in combination.

The cover sheet 4a can be of any material which is liquid permeable and has barrier properties which prevent the inside hydrophilic fiber from protruding. The covering sheet 4a may be, for example, a woven fabric, a non-woven fabric or a perforated plastic sheet.

The non-woven fabric can be formed by using any dry type process (carding process, spun bond process, melt blown process, air-laid process, and the like), wet type process, and the like. Alternatively, these processes may be used in combination. Also, the non-woven fabric may be bonded by thermal bonding, needle punch, chemical bonding, and the like. Alternatively, a spun lace formed in a sheet by a hydroentangling process may be used as the non-woven fabric.

### 2.5. Deformed State

A deformed state of the sanitary napkin 1 and the absorbent body 4 will be described with reference to Fig. 5.

The sanitary napkin 1 can be deformed along with a shape of an intended wearer thereof. Specifically, the sanitary napkin 1 can be deformed along with a shape of a crotch portion of a wearer's body when applied thereto. In other words, as the absorbent body 4 deforms along with the shape of the crotch portion (see Fig. 5), the sanitary napkin 1 also deforms along with the shape of the crotch portion.

As shown in Fig. 5, the absorbent body 4 curves into an arched shape (curved surface shape) in the longitudinal direction Y, with the surface sheet 2 side facing inside. In addition, in the width direction X, the front edge portion 40F has a planar shape. From a central portion in the longitudinal direction Y to the rear edge portion 40R, a central portion in the width direction X projects to the surface sheet 2 side. The deformed state of the absorbent body 4 of the sanitary napkin 1 when in the worn state will be further described hereinafter.

First, the front edge portion 40F of the absorbent body 4 is folded to be raised toward the surface sheet 2 side at each of the front bending portions 10, 10. Also, the rear edge portion 40R of the absorbent body 4 is folded to be raised toward the surface sheet 2 side at each of the rear bending portions 11, 11.

A face of the back sheet 2 side in the second region 20 formed between the front bending portions 10, 10 and the rear bending portions 11, 11 which works as a point of folding, is deformed in the longitudinal direction Y into a planar shape or a slightly curved shape, and is deformed in the width direction X so as to narrow the distance between both ends in the width direction X. Specifically, when forces F1, F1 from the thighs is applied from both sides in the width direction X to a central portion in the longitudinal direction Y of the sanitary napkin 1, the absorbent body 4 is deformed so as to narrow the distance between both ends in the width direction X thereof. In this way, as the absorbent body 4 is deformed in the width direction X so as to narrow the distance between both ends in the width direction, the second region 20 is deformed to project (be raised) toward the surface sheet 2 (a skin contacting side).

Here, the second region 20 is raised toward the surface sheet 2 (the skin contacting side) even in a not-worn state, so as to be appropriately brought into contact with a concave portion and a groove portion of wearer's body at first. The second region 20 is even more appropriately brought into contact with a concave portion and a groove portion of wearer's body by being deformed so as to project toward the surface sheet 2 (the skin contacting side) as described above. In other words, in a worn state, the sanitary napkin 1 is appropriately brought into contact with a concave portion and a groove portion of wearer's body. Specifically, in a worn state, the sanitary napkin 1 is appropriately brought into contact with the vaginal opening, the excretory portion and the like in the crotch portion.

Subsequently, a region from the first bending portions 12, 12 to the rear edge portion 40R of the absorbent body 4 is deformed to be folded to the back sheet 3 side (non-skin contacting side), at each of the first bending portions 12, 12 as a point of folding. Thus, the first region 21, which is formed between the rear bending portions 11, 11 and the first bending portions 12, 12, is deformed to project (be raised) toward the surface sheet 2 (the skin contacting side).

Additionally, since the second bending portion 13 which is formed in the first region 21 is the point of folding, the projected first region 21 has a shape projecting toward the surface sheet 2 side (skin-contacting surface side) at a position of the second bending portion 13. The first region 21 is raised so as to fit a concave shape from the vaginal opening to the anus. In other words, in a worn state, the sanitary napkin 1 is appropriately brought into contact with a groove portion and a concave portion from the vaginal opening to the anus on the crotch.

Additionally, a region at the side of the rear edge portion 40R of the absorbent body 4 is folded at the third bending portions 14, 14 so that both sides in the width direction X project toward the back sheet 3 side (non-skin contacting side). In other words, a region at the side of the rear edge portion 40R in the absorbent body 4 is deformed so that a central portion in the width direction X projects toward the back sheet 2 side (non-skin contacting side). By deforming in this way, a region between one and another of third bending portions 14, 14 is disposed along a groove portion of the buttocks. In other words, the sanitary napkin 1 is disposed so as to follow along a groove portion of the buttocks in a worn state. Here, the third bending portions 14, 14 serve to maintain a projecting deformed state of the central portion in the width direction X toward the wearer's body.

In addition, a region from the fourth bending portions 15, 15 to the front edge portion 40F of the absorbent body 4 bends at points of folding which are the fourth bending portions 15, 15 toward the surface sheet 2. Thus, the absorbent body 4 deforms so as to fit the shape of the crotch portion. In other words, the sanitary napkin 1 deforms so as to fit the shape of the crotch portion.

As described above, in the state in which the sanitary napkin 1 is worn, the absorbent body 4 deforms so as to fit the shape of the wearer's body. In other words, when worn, the sanitary napkin 1 deforms so as to follow along a shape of the wearer's body.

### 2.6. Manufacturing Method

A manufacturing method of the absorbent body 4 is described hereinafter with reference to Figs. 6 and 7.

A manufacturing method of the core 4B is mainly described. As shown in Fig. 6, a manufacturing device 900 forming the core 4B comprises: a cylindrical suction drum 920; a plurality of mesh cases 200 arranged along the circumference of the suction drum 920; a scuffing roller 930 for scraping absorbent material such as pulp, polymer and the like which is stacked in the mesh cases 200; an opening unit 910 for opening a pulp sheet reeled out from a rolled pulp sheet 901; and a conveyer 950 for conveying in a prescribed direction both tissue 904 which is reeled out from rolled tissue 903 and the core 4B which is formed in the mesh case 200.

As shown in Fig. 7, the mesh case 200 for forming the core 4B of a predetermined shape has a wall portion surrounding the outer periphery with a predetermined height, and a planar bottom portion surrounded by the wall. On this bottom portion, a plurality of convex portions 210 projecting to a same side as the wall portion formed, and a concave portion 220 concave toward the other side, are formed.

As shown in Fig. 7, each of the plurality of convex portions 210 is formed in a position corresponding to a bending portion. In the case that an absorbent material is stacked in the mesh case 200, the absorbent material is not stacked in the plurality of convex portions 210, so each of the bending portions are thus formed in positions corresponding to the convex portions 210. Here, the shape of the convex portions 210 is a rectangular cross section in a planar direction, and a tapered trapezoidal cross section in a direction vertical to the planar direction.

Additionally, as shown in Fig. 7, the concave portion 220 is formed in a position corresponding to the second region.
The absorbent material is stacked in the concave portion 220 and forms the second region raised (projecting) toward the surface sheet 2 (a lower surface of Fig. 7).

With reference to Fig. 6, a manufacturing method for the absorbent body 4 is described. First, a pulp sheet reeled out from a rolled pulp sheet 901 is opened by the opening unit 910. The opened pulp 902 which is opened is stacked on the circumference of the suction drum 920 so as to be disseminated. Since the mesh cases 200 are disposed on the circumference of the suction drum 920, the opened pulp 902 is stacked on the mesh cases 200. Specifically, the opened pulp 902 is not stacked in the convex portion 210 of the mesh container 200, but the opened pulp 902 is stacked in all the other areas.

Next, the suction drum 920 rotates to bring the mesh cases 200 to a lower position in Fig. 6. Then, the opened pulp 902 stacked on top of the convex portions 210 and an opened pulp 902 excessively stacked on the mesh cases 200 is scraped away by the scuffing roller 930. Thus, a bottom face and the opposite face in the mesh cases 200 in a core portion 4b are formed so as to be planar. In addition, the bending portions, where the opened pulp 902 substantially is not present, are formed.

Subsequently, as the mesh cases 200 are brought to the further lower position in Fig. 6, the core 4b of a predetermined shape is transferred (disposed) on the tissue 904 which is reeled out from the rolled tissue 903. Here, since the convex portion 210 has a tapered trapezoidal cross section in a direction vertical to the planar direction, the core 4b is smoothly transferred (disposed) onto the tissue 904 without losing its shape. Additionally, a polymer is blended with the core portion 4B in a predetermined phase.

The conveyer 950 conveys the tissue 903, with the core 4b transferred (disposed) thereto, toward a predetermined direction. Finally, by means of a device not shown herein, the tissue 903 is cut into a predetermined shape and deformed so as to cover the core 4b. The absorbent body 4 is thus manufactured.

All or a portion of the convex portions 210 of the mesh case 200 are preferably not air permeable. For example, the convex portions 210 are preferably made non-air permeable, by means of: the mesh of the convex portions 210 being blocked by resin; a surface of the convex portions 210 being blocked by a tape, a metal plate, and the like; and the convex portions 210 being formed of material such as rubber which is not air permeable.

The convex portions 210 have a length in the longitudinal direction in the range of 5 to 50 mm, and preferably in the range of 10 to 30 mm; and a length in the width direction (width) in the range of 1 to 10 mm, and preferably in the range of 2 to 5 mm. A height of the convex portions 210 can suitably be determined in accordance with a weight of the absorbent body 4. For example, the height is in the range of 1 to 20 mm in cases in which the weight of pulp or polymer is in the range of 50 to 2000 g/m², in the range of 1 to 5 mm in cases in which the weight of pulp or polymer is in the range of 50 to 800 g/m².

### 2.7. Effect

According to the present embodiment, the sanitary napkin 1 deforms so as to fit the shape of the wearer's body, so as to be appropriately brought into contact with the vaginal opening, the anus and the like. Thus, the sanitary napkin 1 appropriately absorbs excretory substances such as menstrual blood.

Furthermore, since the sanitary napkin 1 of the present embodiment has the second region 20 which is raised toward the wearer's body, a position corresponding to the second region 20 on the surface sheet 20 of the sanitary napkin 1 is appropriately brought into contact with the excretory part such as the vaginal opening. Thus, the sanitary napkin 1 appropriately absorbs the menstrual blood and the like discharged from the vaginal opening. In addition, in the worn state, since the second region 20 is deformed so as to be raised toward the surface sheet 2 and the wearer's body, a position corresponding to the second region 20 on the surface sheet 2 of the sanitary napkin 1 is more appropriately brought into contact with the vaginal opening. Thus, the sanitary napkin 1 absorbs more appropriately the menstrual blood discharged from the vaginal opening.

Moreover, since the sanitary napkin 1 of the present embodiment, in the worn state, has the first region which deforms so as to be raised toward the surface sheet 2 side which is the wearer's body side, a position corresponding to the first region on the surface sheet 2 of the sanitary napkin 1 is brought into contact with, and follows, the groove portion from the vaginal opening to the anus. Leakage of menstrual blood running along the skin of the wearer and from the skin-contacting surface of the sanitary napkin 1 is thus inhibited.

Furthermore, the sanitary napkin 1 of the present embodiment has the central portion in the width direction X, in the region from the first region 21 to the rear edge portion 20R, deform so as to be raised toward the surface sheet 2 which is the wearer's body. Thus, a region in the proximity of the rear edge portion 40R of the sanitary napkin 1 deforms so as to be in contact with the groove portion of the wearer's body, so as to inhibit leakage of menstrual blood and the like running along the groove portion in the buttocks.

Moreover, since the sanitary napkin 1 of the present embodiment does not have a bending portion formed in the second region 20, which is a region in contact with the vaginal opening and the like, an unexpected deformation in the first region 21 does not easily occur. Thus, the sanitary napkin 1 can inhibit leakage of menstrual blood and the like due to an unwanted deformation thereof.

### 3. Other embodiments and components

### 3.1. Other Embodiments

As above, a preferred embodiment of the present invention has been described, but it is noted that the present invention is not limited to the embodiment described above, and that many other embodiments are possible. For example, in order for the sanitary napkin 1 to deform more easily along the shape of the wearer's crotch portion, embossing patterns reflecting the crotch shape or compressed grooves (so-called hinge portion, anti-leakage grooves) can be formed. Here, the compressed grooves and embossing patterns can be formed on both the surface sheet 2 and the absorbent body 4, or formed only on the absorbent body 4.

Here, the embossing patterns can be formed on the absorbent body 4 so as to inhibit deformation or twist thereof, or to adjust the thickness thereof.

The embossing patterns can be formed on the absorbent body 4 by inserting the absorbent body 4 between a patterned embossing roller and a flat roller. The pattern of this embossing roller can be selected from lattice pattern, dots, a corrugated pattern, and the like. Here, in order to prevent the embossed portion from entering the bending portion and thus breaking the cover sheet, a lattice pattern is preferred.

Additionally, to avoid the side leakage of liquid such as the menstrual blood, a gather of elastic material and the like may be provided on both sides in the width direction X of the sanitary napkin 1.

Although, in the present embodiment, using the mesh cases 200 formed by the convex portions 210, substantially the binding portions in which are not disposed any absorbent material are formed, but the present invention is not limited thereto; for example, the bending portions can also be formed by forming a core of a uniform weight and thickness, and then partially punching the core with a predetermined pattern. Specifically, the bending portions with no absorbent material can be formed by inserting the absorbent body between a roll disposed in a predetermined pattern with slit blades with a predetermined shape and of a predetermined width, and a flat roll.

In the present embodiment, the bending portions 210 have a rectangular cross section in a direction vertical to the planar direction; however, the cross section can be of a tapered trapezoid, diamond shape, triangle and the like.

Additionally, in the present embodiment, the sanitary napkin 1 is described as an example of an absorbent article; however, the present invention is not limited to the above, and may include, as an absorbent article, for example: auxiliary pads for sanitary napkin; panty liners; disposable diapers for infants; disposable diapers for adults; and urine absorbing pads.

## Claims

1. An absorbent article having a width direction and a longitudinal direction orthogonal to the width direction, and
a front edge portion and a rear edge portion in the longitudinal direction, comprising:
a surface sheet such that at least a portion is liquid permeable;
a back sheet which is liquid impermeable; and
an elongated shaped absorbent body disposed between the surface sheet and the back sheet along the longitudinal direction,
wherein the absorbent body has a bending portion with a bending stiffness lower than an average bending stiffness of the absorbent body and lower than a bending stiffness of neighboring regions thereof, and
wherein the bending portion comprises:
a front bending portion disposed at a front portion in the longitudinal direction, formed to extend from a central side in the width direction to an external side in the width direction as the front bending portion extends from the front edge portion side to a central side in the longitudinal direction;
a rear bending portion disposed at a rear portion in the longitudinal direction, formed to extend from the central side in the width direction to the external side in the width direction as the rear bending portion extends from the rear edge portion side to the central side in the longitudinal direction; and
a first bending portion formed between the rear bending portion and the rear edge portion, formed to extend from the external side in the width direction to the central side in the width direction, as the first bending portion extends from the rear edge portion side to the central side in the longitudinal direction.

2. The absorbent article according to claim 1, wherein the absorbent body further comprises:
a first region which can be raised toward the surface sheet side while the absorbent body is in the worn state, formed between the rear bending portion and the first bending portion; and
a second bending portion formed in the first region.

3. The absorbent article according to claim 1 or 2,
wherein the bending portion further comprises a third bending portion formed at both sides of a first center line dividing equally the absorbent body in the width direction, extending in the longitudinal direction between the first bending portion and the rear edge portion.

4. The absorbent article according to any one of claims 1 to 3, wherein the bending portion further comprises a fourth bending portion formed to extend from the central side in the width direction to the external side in the width direction as the fourth bending portion extends from the front edge portion side to the central side in the longitudinal direction between the front bending portion and the front edge portion.

5. The absorbent article according to any one of claims 1 to 4, wherein the bending portion is formed substantially symmetrically with respect to the first center line.
